# EUROPEAN PATENT APPLICATION

(11) **EP 4 635 526 A1**
(43) Date of publication of application: **22.10.2025**
(21) Application number: 23903864.9
(22) Date of filing: 04.12.2023
(51) Int. Cl.: A61L 27/36, C09D 11/04, B33Y 70/00

(54) **DEVICE FOR MANUFACTURING BIOINK**

(30) Priority: 16.12.2022 KR 20220177590
(71) Applicant: POSCO Holdings Inc., Pohang-si, Gyeongsangbuk-do 37859 (KR); RESEARCH INSTITUTE OF INDUSTRIAL SCIENCE & TECHNOLOGY, Pohang-si, Gyeongsangbuk-do 37673 (KR); POSTECH Research and Business Development Foundation, Pohang-si, Gyeongsangbuk-do 37673 (KR)
(72) Inventor: LEE, Boram, Busan 48113 (KR); KIM, Hyejin, Pohang-si, Gyeongsangbuk-do 37673 (KR); JANG, Jinah, Pohang-si, Gyeongsangbuk-do 37673 (KR)
(74) Representative: Pfenning, Meinig & Partner mbB
(86) International application number: PCT/KR2023/019820
(87) International publication number: WO 2024/128661

(57) **Abstract**

A bio-ink manufacturing device according to the present disclosure is provided. The bio-ink manufacturing device include: a reaction vessel for performing a reaction on tissues of decellularized extracellular matrix and an ink solution; a bio-ink liquefy portion for stirring and compacting the tissues of decellularized extracellular matrix and liquefying the same into bio-ink; and a bio-ink discharge portion discharging the bio-ink whose liquefaction is completed to an outside of the reaction vessel.

## Description

### [Technical Field]

The present disclosure relates to a bio-ink manufacturing device.

### [Background Art]

As the global aging trend continues, the number of people with chronic diseases is increasing, and interest is focused on artificial organ manufacturing technology using 3D bioprinting.

Bio-ink is a term referring to an ink material for outputting artificial organs using a 3D bio printer, and is manufactured based on an extracellular substrate obtained after decellularizing the extracellular matrix.

To manufacture the artificial organs, a large amount of bio-ink is needed, but the technology to mass-produce bio-ink has not been developed, making it difficult to supply the necessary amount in proportion to demand. As a result, research utilizing bio-ink has difficulty in achieving high scalability.

There is no facility for digesting a large amount of decellularized extracellular matrix (dECM) tissues when making a solution of bio-ink, and a small amount of ink (e.g., 8.4 ml) is manufactured in a conical tube, so the properties of bio-ink are not uniform for each tube, which was raised as an issue in commercialization.

When manufacturing the bio-ink to meet consumer needs, the viscosity of the bio-ink may increase to hundreds of thousands of cP due to the high concentration of decellularized extracellular matrix organs contained in the ink, but a device for properly dispensing the bio-ink after manufacturing it has not been designed.

### [DETAILED DESCRIPTION OF THE INVENTION]

### [Technical Problem]

The present disclosure attempts to provide a bio-ink manufacturing device for liquefying a large amount of decellularized extracellular matrix (dECM) tissues when manufacturing bio-ink in normal concentration (1% dECM), and liquefying bio-ink with high viscosity and quantitatively discharging the same from a same reaction vessel when manufacturing bio-ink in high concentration.

### [Technical Solution]

An embodiment of the present disclosure provides a device for manufacturing bio-ink including: a reaction vessel for performing a reaction on tissues of decellularized extracellular matrix (dECM) and an ink solution so as to manufacture bio-ink; and a bio-ink liquefy portion for stirring and compacting the tissues of decellularized extracellular matrix in the reaction vessel and liquefying the same into bio-ink.

The device for manufacturing bio-ink may include a bio-ink discharge portion spaced apart from the bio-ink liquefy portion by a predetermined distance, and discharging the bio-ink whose liquefaction is completed in the reaction vessel to an outside of the reaction vessel.

The device for manufacturing bio-ink may include a move portion for moving the reaction vessel in which liquefaction of bio-ink is completed to the bio-ink discharge portion from the bio-ink liquefy portion.

The reaction vessel may have a cylindrical shape having an opened upper portion and having a space for storing the tissues of decellularized extracellular matrix (dECM).

An internal side of the reaction vessel may be coated with a material having chemical resistance to the tissues of decellularized extracellular matrix and an ink solution.

The reaction vessel may have a double jacket shape including an inner tube and an outer tube.

A coolant injecting hole for injecting a temperature control coolant to a space between the inner tube and the outer tube, and a coolant outlet for discharging the injected coolant to an outside of the outer tube may be installed in the outer tube.

The bio-ink liquefy portion may include a first cover arranged on an upper portion of the reaction vessel and covering an upper end of the reaction vessel; and a rotation shaft rotatably combined to an upper end of the first cover in a vertical direction and protruding to a lower portion of the first cover by a predetermined length.

The bio-ink liquefy portion may include an impeller combined to a lower end of the rotation shaft, and stirring and compacting the tissues of decellularized extracellular matrix and the ink solution injected into the reaction vessel.

The impeller may be manufactured or is coated with a material that has chemical resistance to the tissues of decellularized extracellular matrix and the ink solution.

A driving motor combined to an upper end of the rotation shaft and rotating the rotation shaft may be installed on an upper portion of the first cover.

At least one solution injection hole port for injecting the ink solution may be installed in the first cover.

A PH measuring device for measuring a hydrogen ion concentration exponent (PH) in the reaction vessel may be installed in the first cover.

An internal observation camera for observing an inside of the reaction vessel may be installed in the first cover.

A first lift portion for lifting the first cover in the vertical direction may be combined to the first cover.

The first lift portion may include a first combination member combined to the first cover in a lifting way, and a first lift manipulating portion combined to an end of the first combination member and lifting the first combination member.

The move portion may include a support plate installed on a lower end of the reaction vessel and supporting the reaction vessel; at least one sliding duct combined to a lower end of the support plate; and a sliding rail installed in a length direction of a base frame between the base frame and the support plate and on which the sliding duct is moveably combined.

The sliding duct may be formed of a cylindrical duct of which an inside is empty.

A fixing lever for stopping movement of the sliding duct may be combined to the sliding duct.

The sliding rail may be arranged in parallel to the base frame.

A first support frame and a second support frame may be vertically installed at respective ends of the base frame, and the sliding rail may be connected between the first support frame and the second support frame.

The bio-ink discharge portion may include a second cover arranged on an upper portion of the reaction vessel and covering an upper end of the reaction vessel; and a syringe piston arranged vertical on a lower end of the second cover, and sealing the inside of the reaction vessel and lifting vertically.

The bio-ink discharge portion may include a bio-ink discharge duct installed on one surface of the reaction vessel, and discharging the bio-ink of which liquefaction in the reaction vessel is completed to an outside by lowering of the syringe piston.

A servomotor for lifting the syringe piston may be combined to the second cover.

A second lift portion for vertically lifting the servomotor may be combined with the servomotor.

The second lift portion may include a second combination member combined to the servomotor in a lifting way, and a second lift manipulating portion combined to an end of the second combination member for lifting the second combination member.

A discharge duct control valve for controlling opening/closing of the bio-ink discharge duct may be installed in the bio-ink discharge duct.

A discharged amount of the bio-ink discharged from the discharge duct control valve may be controlled by controlling an operation of the servomotor by a controller connected to the servomotor.

### [Advantageous Effects]

According to the embodiment of the present disclosure, a large amount of decellularized extracellular matrix (dECM) tissue may be liquefied when manufacturing the bio-ink in normal concentration (1% dECM), and the bio-ink with high viscosity may be liquefied and may be quantitatively discharged from the same reaction vessel when manufacturing the bio-ink in high concentration.

That is, the reaction vessel in which liquefaction of bio-ink is completed may be moved to the bio-ink discharge portion by the move portion, and the bio-ink may be discharged from the bio-ink discharge portion, thereby preventing the loss caused by moving bio-ink.

The large amount of decellularized extracellular matrix (dECM) tissues may be efficiently liquefied and may be efficiently discharged, and particularly, homogeneity may be obtained in estimating the properties among vials when liquefying bio-ink, thereby contributing to commercialization and industrialization of the bio-ink.

### [Description of the Drawings]

FIG. 1 shows a schematic diagram of a bio-ink manufacturing device according to an embodiment.
FIG. 2 shows a partial lateral view of a bio-ink discharge portion of a bio-ink manufacturing device according to an embodiment.
FIG. 3 shows a photograph on a process for inputting sterilized pig's placenta decellularized extracellular matrix powder and liquefy solution to a reaction vessel using a bio-ink manufacturing device according to an embodiment.
FIG. 4 shows a photograph on monitoring a process for liquefying a sterilized pig's placenta decellularized extracellular matrix using a bio-ink manufacturing device with respect to a liquefying time according to an embodiment where T represents time.
FIG. 5 shows a sol-gel test photograph on a process for digesting a sterilized pig's placenta decellularized extracellular matrix using a bio-ink manufacturing device according to an embodiment, where (a) shows a photograph in a digestion state, (b) shows a photograph in a gel state, and (c) shows a graph on a viscosity measuring result with respect to shear rates.

### [BEST MODE FOR INVENTION]

The present disclosure will be described more fully hereinafter with reference to the accompanying drawings, in which embodiments of the disclosure are shown. As will be readily appreciated by one of ordinary skill in the art to which the present disclosure belongs, embodiments described below may be modified in various forms without departing from the concept and scope of the present disclosure. Wherever possible, the same or similar parts are denoted using the same reference numerals in the drawings.

The terminologies used herein are used just to illustrate a specific embodiment, but are not intended to limit the present disclosure. It must be noted that, as used in the specification and the appended claims, the singular forms used herein include plural forms unless the context clearly dictates the contrary. It will be further understood that the term "comprises" or "includes", used in this specification, specifies stated properties, regions, integers, steps, operations, elements, and/or components, but does not preclude the presence or addition of other properties, regions, integers, steps, operations, elements, components, and/or groups.

All terms including technical terms and scientific terms used herein have the same meaning as the meaning generally understood by a person with ordinary skill in the art to which the present disclosure pertains. The terminologies that are defined previously are further understood to have the meaning that coincides with relating technical documents and the contents that are disclosed currently, but are not to be interpreted as the idealized or very official meaning unless so defined.

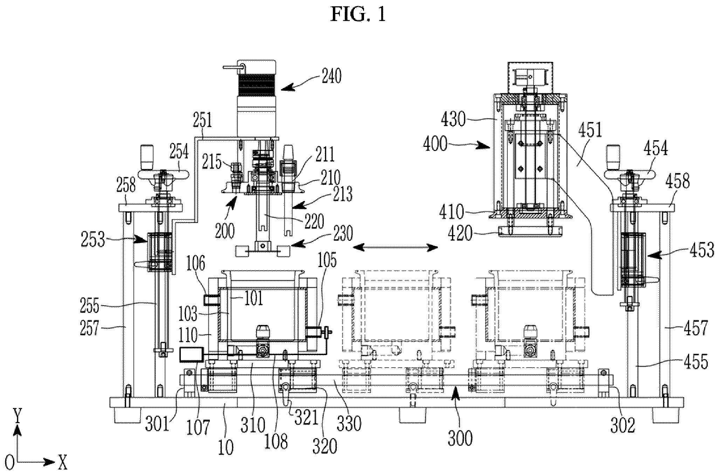

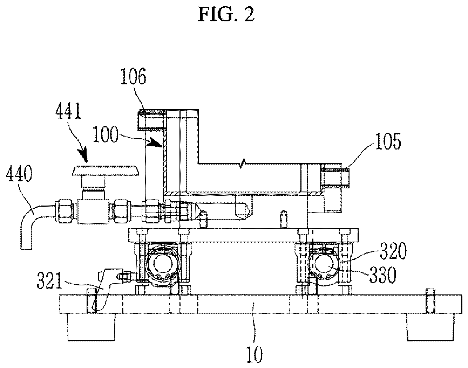

FIG. 1 shows a schematic diagram of a bio-ink manufacturing device according to an embodiment, and FIG. 2 shows a partial lateral view of a bio-ink discharge portion of a bio-ink manufacturing device according to an embodiment.

Referring to FIG. 1 and FIG. 2, the bio-ink manufacturing device may include a reaction vessel 100, a bio-ink liquefy portion 200, a move portion 300, and a bio-ink discharge portion 400.

The reaction vessel 100 may perform a reaction between the tissue of decellularized extracellular matrix (dECM) and the ink solution for producing bio-ink.

The bio-ink liquefy portion 200 may liquefy the tissue of the decellularized extracellular matrix stored in the reaction vessel 100 into bio-ink by stirring and compacting it.

The bio-ink discharge portion 400 may be disposed at a predetermined distance from the bio-ink liquefy portion and may discharge bio-ink, which has completed liquefaction within the reaction vessel 100, to the outside of the reaction vessel 100.

The move portion 300 may move the reaction vessel 100, in which the liquefaction of the bio-ink is completed, to the bio-ink discharge portion 400 along a first direction (X direction of FIG. 1) from the bio-ink liquefy portion 200.

The reaction vessel 100 may be movably coupled to the move portion 300 and may be moved to the bio-ink discharge portion 400 from the bio-ink liquefy portion 200 along the first direction (X direction of FIG. 1) by the move portion 300.

The reaction vessel 100 may be formed in a cylindrical shape, such as having an opened upper portion to allow tissues of the decellularized extracellular matrix (dECM) to be injected and having a space for storing the tissues of the decellularized extracellular matrix (dECM) therein.

An inner surface of the reaction vessel 100 may be coated with a material such as Teflon that has chemical resistance to the tissues of the decellularized extracellular matrix and ink solutions.

The reaction vessel 100 may have a double jacket shape formed with an inner tube 101 and an outer tube 103, to control the temperature of the process for liquefying the tissue of the decellularized extracellular matrix.

The outer tube 103 may be provided with a coolant injecting hole 105 for injecting a coolant for controlling temperatures into the space between the inner tube 101 and the outer tube 103, and a coolant outlet 106 for discharging the injected coolant to the outside of the outer tube 103.

The coolant injecting hole 105 may be connected to the cooling device 107 by a connecting pipe 108.

An insulation material 110 may be installed on an outside of the reaction vessel 100 to surround the reaction vessel 100 and insulate the reaction vessel 100.

The bio-ink liquefy portion 200 may include a first cover 210, a rotation shaft 220, and an impeller 230.

The first cover 210 may be placed on an upper portion of the reaction vessel 100 and may cover an upper end of the reaction vessel 100.

The rotation shaft 220 may be rotatably combined to an upper end of the first cover 210 in the vertical direction (Y direction in FIG. 1), and may protrude to the first cover 210 by a predetermined length.

The impeller 230 may be combined to a lower end of the rotation shaft 220, and may stir and compact the tissues of the decellularized extracellular matrix and the ink solution injected into the reaction vessel 100.

The impeller 230 may be manufactured from a material that has chemical resistance to the tissues of the decellularized extracellular matrix and the ink solutions, or may be coated with a material such as Teflon.

The impeller 230 may have any types of shapes that may easily generate turbulence inside the reaction vessel 100 when rotated by the rotation shaft 220, and may implement a compaction function by friction with an interior wall of the reaction vessel 100.

The impellers 230 may be installed at predetermined intervals on a connecting frame (not shown) that rotatably connects the impellers 230 to the rotation shaft 220.

Therefore, the impellers 230 may move in the direction toward the inner surface of the reaction vessel 100 or toward the center of the reaction vessel 100 due to the free play generated by the centrifugal force when the rotation shaft 220 is rotated, so that the compaction function of the extracellular matrix tissue may be effectively implemented as a function capable of frictionally applying the inner surface of the reaction vessel 100. Hence, when the rotation shaft 220 rotates, a gap may be generated in the impeller 200 by a centrifugal force, and the impeller 200 may move in a direction proceeding to an inside of the reaction vessel 100 or a direction proceeding to a center of the reaction vessel 100, so a function of compacting the extracellular matrix tissue may be effectively implemented with a function of rubbing the internal surface of the reaction vessel 100.

A driving motor 240 combined to an upper end of the rotation shaft 220 and rotating the rotation shaft 220 at a predetermined rate may be installed on an upper portion of the first cover 210.

The driving motor 240 may control the rotation shaft 220 at the rotation rate of, for example, 0 to 500 rpm.

The driving motor 240 may be formed of a magnetic type motor, etc., to easily rotate the rotation shaft 220.

At least one or more solution injection hole ports 211 for injecting an inking solution for inking the tissues of the decellularized extracellular matrix may be installed in the first cover 210.

A PH measuring device 213 for measuring the hydrogen ion concentration exponent (PH) in the reaction vessel 100 may be installed in the first cover 210.

An internal observation camera 215 may be installed in the first cover 210 to observe the inside of the reaction vessel 100 so as to check the process for liquefying the decellularized extracellular matrix tissue within the reaction vessel 100, i.e., whether the decellularized extracellular matrix tissue is digested.

The first cover 210 may be combined with a first lift portion 250 for lifting the first cover 210 in the vertical direction (Y direction in FIG. 1) to thereby combine or separate the first cover 210 with/from the reaction vessel 100.

The first lift portion 250 may include a first combination member 251 and a first lift manipulating portion 253.

The first combination member 251 may be integrally combined to the first cover 210 in a lifting way.

The first lift manipulating portion 253 may be connected to one end of the first combination member 251 and may be spaced apart from the reaction vessel 100, and may lift the first combination member 251.

The first lift manipulating portion 253, as shown in FIG. 1, may be controlled manually or automatically (not shown).

When the first lift manipulating portion 253 is a manual control type, a first manipulation wheel 254 for manually manipulating the upper end of the first lift manipulating portion 253 may be combined thereto.

That is, the first lift manipulating portion 253 may be lifted along the first support shaft 255 by rotating the first manipulation wheel 254.

When the first lift manipulating portion 253 is an automatic control type, it may be made of a motor or an air cylinder (not shown).

The first support shaft 255 may be supported by a first vertical frame 257 installed in the vertical direction (Y direction of FIG. 1) at a predetermined interval on a base frame 10, and a first horizontal frame 258 combined to an upper end of the first vertical frame 257 in the vertical direction (X direction of FIG. 1).

The base frame 10 may be placed in the length direction (X direction in FIG. 1) on an installation surface or a floor surface.

The move portion 300 may include a support plate 310, a sliding duct 320, and a sliding rail 330.

The support plate 310 may be installed at the lower end of the reaction vessel 100 and may support the reaction vessel 100.

At least one sliding duct 320 may be combined to a lower end of the support plate 310.

The sliding rail 330 may be installed in the length direction of the base frame 10 between the base frame 10 and the support plate 310, and the sliding duct 320 may be movably combined thereto.

The sliding duct 320 may be formed of a cylindrical duct or the like with an empty interior so that it may be easily moved along the sliding rail 330.

At least one sliding duct 320 may be coupled with a fixing lever 321 for stopping the movement of the sliding duct 320 along the sliding rail 330.

The sliding rail 330 may be placed in parallel to the base frame 10 for easy movement of the reaction vessel 100.

A first support frame 301 and a second support frame 302 may be installed in the vertical direction (Y direction in FIG. 1) at both ends of the base frame 10, and a sliding rail 330 may be connected between the first support frame 301 and the second support frame 302.

The bio-ink discharge portion 400 may include a second cover 410, a syringe piston 420, a servomotor 430, and a bio-ink discharge duct 440.

The second cover 410 may be placed on the upper portion of the reaction vessel 100 and may cover the upper end of the reaction vessel 100.

The syringe piston 420 may be disposed at the lower end of the second cover 410 in the vertical direction (Y direction in FIG. 1), and may be lifted vertically while closing and sealing the interior of the reaction vessel 1002.

The servomotor 430 may be combined to an upper portion of the second cover 410 and may lift the syringe piston 420.

The bio-ink discharge duct 440 may be installed on a surface of the reaction vessel 100, and may discharge the bio-ink whose liquefaction is completed in the reaction vessel 100 to a vial (not shown) or the like by lowering the syringe piston 420.

A discharge duct control valve 441 for controlling the opening and closing of the bio-ink discharge duct 440 may be installed in the bio-ink discharge duct 440.

The discharged amount of bio-ink discharged from the discharge duct control valve 441 may be achieved by controlling the operation of the servomotor 430 by a controller (not shown) that is connected to the servomotor 430 and controls the operation of the servomotor 430.

The controller (not shown) may control the servomotor 430 so that the servomotor 430 may effectively discharge the bio-ink even when the viscosity of the bio-ink reaches hundreds of thousands of cP (centi-poise).

The servomotor 430 may be combined with a second lift portion 450 to lift the servomotor 430 in the vertical direction (Y direction in FIG. 1) so as to insert or separate the syringe piston 420 into/from the reaction vessel 100.

The second lift portion 450 may include a second combination member 451 and a second lift manipulating portion 453.

The second combination member 451 may be integrally combined to the servomotor 430 in a lifting way.

The second lift manipulating portion 453 may be combined to an end of the second combination member 451, may be spaced apart from the reaction vessel 100, and may lift the second combination member 451.

The second lift manipulating portion 453, as shown in FIG. 1, may be controlled manually or automatically (not shown).

When the second lift manipulating portion 453 is a manual control type, a second manipulation wheel 454 for manually manipulating the upper end of the first lift manipulating portion 253 may be combined thereto.

That is, the second lift manipulating portion 453 may be lifted along the second support shaft 455 by the rotation of the second manipulation wheel 454.

When the second lift manipulating portion 453 is an automatic control type, it may be made of a motor or an air cylinder (not shown).

The second support shaft 255 may be supported by a second vertical frame 457 installed in the vertical direction (Y direction of FIG. 1) at a predetermined interval on the base frame 10, and a second horizontal frame 458 combined to the upper end of the second vertical frame 457 in the vertical direction (X direction of FIG. 1).

The operation of the bio-ink manufacturing device according to an embodiment will now be described with reference to FIG. 1 and FIG. 2.

To produce bio-ink, when liquefying the bio-ink, the reaction vessel 100 may be disposed in the center of the sliding rail 330, and the decellularized extracellular matrix (dECM) tissues may be input to the lower end of the reaction vessel 100.

The reaction vessel 100 may be disposed to the left side (left side in FIG. 1) from the center of the sliding rail 330 using the move portion 300, that is, on the bio-ink liquefy portion 200, and the movement of the sliding duct 320 may be stopped by fixing the fixing lever 321.

In this state, the first combination member 251 may be vertically lowered using the first lift manipulating portion 253 of the first lift portion 250 to lower the first cover 210 and seal the upper end of the reaction vessel 100.

The ink solution for making the tissue of the decellularized extracellular matrix into ink is injected into the reaction vessel 100 through the solution injection hole port 211 installed in the first cover 210.

The temperature in the reaction vessel 100 may be controlled at a predetermined temperature, and the driving motor 240 may be driven to rotate the rotation shaft 220 at a predetermined rate.

As the rotation shaft 220 rotates, the impeller 230 inserted into the reaction vessel 100 may be rotated to stir and compact the tissue of the decellularized extracellular matrix and the ink solution injected into the reaction vessel 100.

Accordingly, the tissues of the decellularized extracellular matrix and the ink solution may be liquefied into bio-ink by the reaction therebetween.

The bio-ink liquefying process may be observed by checking whether the decellularized extracellular matrix tissue in the reaction vessel 100 is digested using the internal observation camera 215 disposed at the upper portion of the reaction vessel 100.

The reaction vessel 100 may have a double jacket configuration including an inner tube 101 and an outer tube 103 so the interior of the reaction vessel 100 may be controlled at a predetermined temperature required for the process for liquefying the tissues of the decellularized extracellular matrix.

When the liquefaction of bio-ink is completed in the reaction vessel 100, the driving motor 240 may be stopped to stop the rotation of the rotation shaft 220 and the impeller 230, and the first combination member 251 may be vertically lifted using the first lift manipulating portion 253 to lift the first cover 210 and separate the same from the upper portion of the reaction vessel 100 to the upper portion.

The reaction vessel 100, where the liquefaction of bio-ink is completed, may be moved to the bio-ink discharge portion 400 from the bio-ink liquefy portion 200 by the move portion 300.

That is, the fixed combination of the fixing lever 321 is released to release the stopped state of the sliding duct 320, the reaction vessel 100 may be disposed to the right side of the sliding rail 330, that is, the bio-ink discharge portion 400 from the bio-ink liquefy portion 200 by using the move portion 300, and the movement of the sliding duct 320 may be stopped by fastening the fixing lever 321.

In this state, the second combination member 451 may be vertically lowered using the second lift manipulating portion 453 of the second lift portion 450 to lower the second cover 410 so that a lower end of the servomotor 430 may cover the upper end of the reaction vessel 100 and may seal the same.

When the servomotor 430 is operated to lower the syringe piston 420, the bio-ink whose liquefaction in the reaction vessel 100 is completed by the lowering of the syringe piston 420 may be discharged to a vial or the like disposed outside the reaction vessel 100 from the inside of the reaction vessel 100 through the bio-ink discharge duct 440.

The discharge duct control valve 441 may be controlled to be opened, and the discharged amount of bio-ink discharged from the discharge duct control valve 441 may be achieved by controlling the operation of the servomotor 430 by a controller (not shown) connected to the servomotor 430 and controlling the operation of the servomotor 430.

Therefore, it is possible to efficiently liquefy a large amount of the tissues of decellularized extracellular matrix (dECM), efficiently discharge the same, and particularly to ensure homogeneity during the evaluation of properties between vials when liquefying bio-ink.

When discharging of the bio-ink in the reaction vessel 100 is completed, the second combination member 451 may be vertically raised using the second lift manipulating portion 453 of the second lift portion 450 to raise the second cover 410 and space the same to the upper portion of the reaction vessel 100 by a predetermined distance.

The fixed combination of the fixing lever 321 may be released to release the stationary state of the sliding duct 320, the reaction vessel 100 may be moved to the central portion of the sliding rail 330 from the bio-ink discharge portion 400 using the move portion 300 so that the process for liquefying bio-ink, the process for moving a reaction vessel, and the process for discharging bio-ink may be repeated as described above.

### (Example)

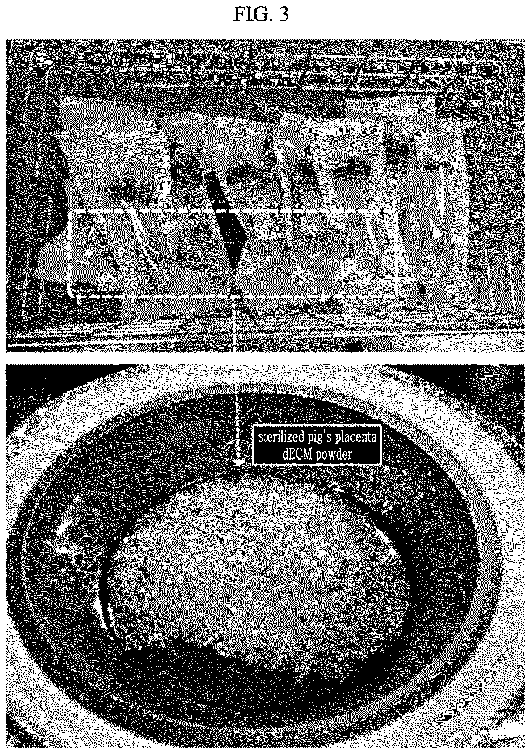

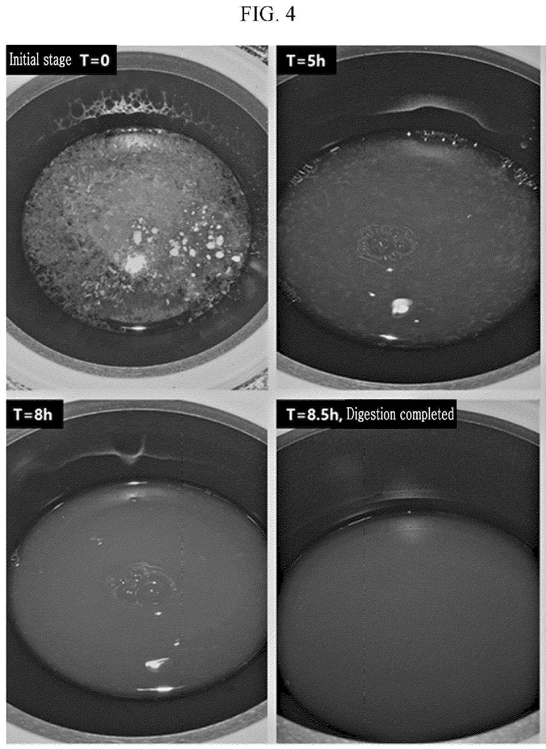

The process for liquefying and discharging bio-ink of sterilized pig's placenta decellularized extracellular matrix (dECM) bio-ink will now be described.
1) As shown in FIG. 3, 5g of sterilized pig placenta decellularized extracellular matrix (dECM) powder, 420 ml of 0.5M acetic acid, and 0.5g of pepsin powder may be injected into the lower end of the reaction vessel, which is the process for manufacturing decellularized extracellular matrix (dECM) bio-ink in concentration of 1%.
2) The lower end of the reaction vessel may be moved to the left end, i.e., the bio-ink liquefy portion, and a manipulator of the first lift manipulating portion may be used to vertically lower a first cover disposed at the upper end of the reaction vessel to close and seal the upper end of the reaction vessel (see FIG. 1), and a correct position of an O-ring at the lower end of the reaction vessel may be checked.
3) The temperature of the double jacket reaction vessel may be controlled by a predetermined temperature of a refrigerated/heating circulator, and for example, it may be set to be 37°C, which is the activation temperature of pepsin. The rotation rate of the impeller inside the reaction vessel may be adjusted to 350 rpm.
4) As shown in FIG. 4, the bio-ink liquefying process may monitor the process for digesting and liquefying the tissue of the decellularized extracellular matrix (dECM) in the reaction vessel by using an internal observation camera installed at the upper end of the liquefied reaction vessel, and in this test, liquefaction is confirmed to be completed in approximately 8.5 hours.
5) Regarding the bio-ink whose liquefaction is complete, the first cover disposed at the upper end of the reaction vessel may be vertically raised using the manipulator disposed at the left side of FIG. 1, and the lower end of the reaction vessel my be moved toward the right end of a sliding rail of the move portion of FIG. 1, i.e., the bio-ink discharge portion. The move portion may be fixed using the fixing lever.
6) The manipulator disposed on the right side of FIG. 1 may be used to vertically lower the servomotor, check the O-ring at the lower end of the reaction vessel, and seal the upper end of the reaction vessel.
7) The bio-ink may be discharged into a vial by lowering the syringe piston using a servomotor controller, or the bio-ink may be discharged using the same method using an HMI system.

### (Test results such as performance, etc.)

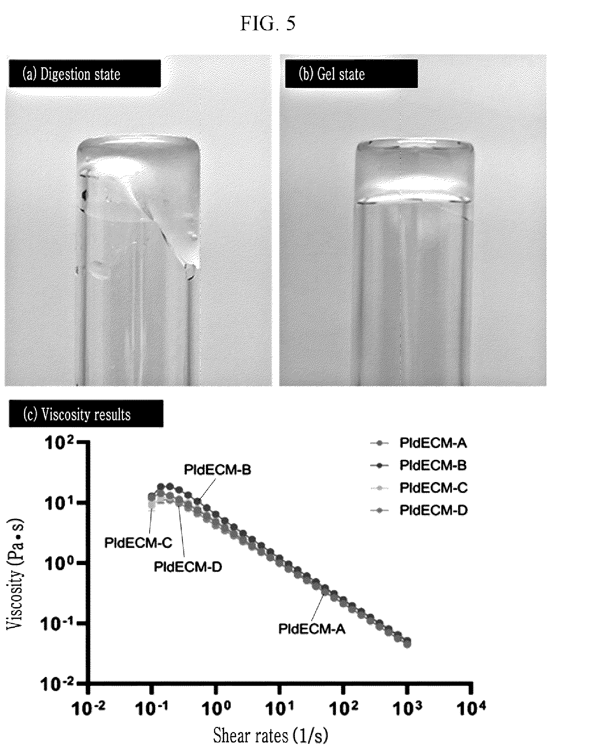

The evidence that the process for digesting the sterilized decellularized extracellular matrix (dECM) is performed well may be supported by the results of a sol-gel test and viscosity measurement, as shown in FIG. 5.

As shown in FIG. 5(a) and FIG. 5(b), it may be confirmed that the ink is well changed to a gel state after 30 minutes of gelation at 37°C and the ink does not fall to the bottom, and the bio-ink is produced in large quantities at the level of 500ml, showing excellent homogeneous viscosity per vial.

While this disclosure has been described in connection with what is presently considered to be practical exemplary embodiments, it is to be understood that the disclosure is not limited to the disclosed embodiments, but, on the contrary, is intended to cover various modifications and equivalent arrangements included within the spirit and scope of the appended claims.

### (Description of symbols)

- 100:: reaction vessel
- 200:: bio-ink liquefy portion
- 300:: bio-ink discharge portion

## Claims

1. A device for manufacturing bio-ink comprising:
a reaction vessel for performing a reaction on tissues of decellularized extracellular matrix (dECM) and an ink solution so as to manufacture bio-ink;
a bio-ink liquefy portion for stirring and compacting the tissues of decellularized extracellular matrix in the reaction vessel and liquefying the same into bio-ink; and
a bio-ink discharge portion spaced apart from the bio-ink liquefy portion by a predetermined distance, and discharging the bio-ink whose liquefaction is completed in the reaction vessel to an outside of the reaction vessel.

2. The device of claim 1, wherein
the device includes a move portion for moving the reaction vessel in which liquefaction of bio-ink is completed to the bio-ink discharge portion from the bio-ink liquefy portion.

3. The device of claim 2, wherein
the reaction vessel has a cylindrical shape having an opened upper portion and having a space for storing the tissues of decellularized extracellular matrix (dECM).

4. The device of claim 3, wherein
an internal side of the reaction vessel is coated with a material having chemical resistance to the tissues of decellularized extracellular matrix and an ink solution.

5. The device of claim 3, wherein
the reaction vessel has a double jacket shape including an inner tube and an outer tube.

6. The device of claim 5, wherein
a coolant injecting hole for injecting a temperature control coolant to a space between the inner tube and the outer tube, and a coolant outlet for discharging the injected coolant to an outside of the outer tube are installed in the outer tube.

7. The device of claim 3, wherein
the bio-ink liquefy portion includes
a first cover arranged on an upper portion of the reaction vessel and covering an upper end of the reaction vessel;
a rotation shaft rotatably combined to an upper end of the first cover in a vertical direction and protruding to a lower portion of the first cover by a predetermined length; and
an impeller combined to a lower end of the rotation shaft, and stirring and compacting the tissues of decellularized extracellular matrix and the ink solution injected into the reaction vessel.

8. The device of claim 7, wherein
the impeller is manufactured or is coated with a material that has chemical resistance to the tissues of decellularized extracellular matrix and the ink solution.

9. The device of claim 7, wherein
a driving motor combined to an upper end of the rotation shaft and rotating the rotation shaft is installed on an upper portion of the first cover.

10. The device of claim 7, wherein
at least one solution injection hole port for injecting the ink solution is installed in the first cover.

11. The device of claim 7, wherein
a PH measuring device for measuring a hydrogen ion concentration exponent (PH) in the reaction vessel is installed in the first cover.

12. The device of claim 7, wherein
an internal observation camera for observing an inside of the reaction vessel is installed in the first cover.

13. The device of claim 7, wherein
a first lift portion for lifting the first cover in the vertical direction is combined to the first cover.

14. The device of claim 13, wherein
the first lift portion includes
a first combination member combined to the first cover in a lifting way, and
a first lift manipulating portion combined to an end of the first combination member and lifting the first combination member.

15. The device of claim 2, wherein
the move portion includes
a support plate installed on a lower end of the reaction vessel and supporting the reaction vessel;
at least one sliding duct combined to a lower end of the support plate; and
a sliding rail installed in a length direction of a base frame between the base frame and the support plate and on which the sliding duct is moveably combined.

16. The device of claim 15, wherein
the sliding duct is formed of a cylindrical duct of which an inside is empty.

17. The device of claim 16, wherein
a fixing lever for stopping movement of the sliding duct is combined to the sliding duct.

18. The device of claim 17, wherein
the sliding rail is arranged in parallel to the base frame.

19. The device of claim 18, wherein
a first support frame and a second support frame are vertically installed at respective ends of the base frame, and
the sliding rail is connected between the first support frame and the second support frame.

20. The device of any one of claim 1 to claim 19, wherein
the bio-ink discharge portion includes
a second cover arranged on an upper portion of the reaction vessel and covering an upper end of the reaction vessel;
a syringe piston arranged vertical on a lower end of the second cover, and sealing the inside of the reaction vessel and lifting vertically; and
a bio-ink discharge duct installed on one surface of the reaction vessel, and discharging the bio-ink of which liquefaction in the reaction vessel is completed to an outside by lowering of the syringe piston.

21. The device of claim 20, wherein
a servomotor for lifting the syringe piston is combined to the second cover.

22. The device of claim 21, wherein
a second lift portion for vertically lifting the servomotor is combined with the servomotor.

23. The device of claim 22, wherein
the second lift portion includes
a second combination member combined to the servomotor in a lifting way, and
a second lift manipulating portion combined to an end of the second combination member for lifting the second combination member.

24. The device of claim 20, wherein
a discharge duct control valve for controlling opening/closing of the bio-ink discharge duct is installed in the bio-ink discharge duct.

25. The device of claim 24, wherein
a discharged amount of the bio-ink discharged from the discharge duct control valve is controlled by controlling an operation of the servomotor by a controller connected to the servomotor.
